# EUROPÄISCHE PATENTSCHRIFT

(11) **EP 4 056 990 B1**
(45) Veröffentlichungstag und Bekanntmachung des Hinweises auf die Patenterteilung: **08.10.2025**
(21) Anmeldenummer: 21161262.7
(22) Anmeldetag: 08.03.2021
(51) Int. Cl.: G01N 21/64, A61P 31/04, G01N 33/58, C12Q 1/18, B01L 3/00

(54) **VERFAHREN ZUR RESPIROMETRISCHEN BESTIMMUNG DER ANTIBAKTERIELLEN WIRKSAMKEIT ANTIBIOTISCHER SUBSTANZEN MITTELS AUF LUMINESZENZ BASIERENDER OXYMETRIE**
METHOD FOR RESPIROMETRIC DETERMINATION OF THE ANTIBACTERIAL EFFICACY OF ANTIBIOTIC SUBSTANCES USING LUMINESCENCE BASED OXYMETRY
PROCÉDÉ DE DÉTERMINATION RESPIROMÉTRIQUE DE L'EFFICACITÉ ANTIBACTERIENNE DES SUBSTANCES ANTIBIOTIQUES AU MOYEN DE L'OXYMÉTRIE À BASE LUMINESCENTE

(43) Veröffentlichungstag der Anmeldung: 14.09.2022
(73) Patentinhaber: Biomimetics Technologies Inc., Toronto, ON M6S 2X4 (CA); Colibri Photonics GmbH, 14473 Potsdam (DE)
(72) Erfinder: Dr. med. Gille, Christoph, 13467 Berlin (DE); Dr. Schmälzlin, Elmar, 14473 Potsdam (DE); Israelowitz, Meir, Toronto, Ontario M6S 2X4 (CA); von Schroeder, Herbert P., Toronto, Ontario M6S 2X4 (CA); Holm, Chris, 42105 Wuppertal (DE); Rizvi, Syed, Oviedo, FL Florida 327765 (US)
(74) Vertreter: Jabbusch, Matthias

(56) Entgegenhaltungen:
- WO-A1-2013/053381
- US-A1- 2008 078 257
- HALASA R ET AL: "Comparison of fluorescence optical respirometry and microbroth dilution methods for testing antimicrobial compounds", JOURNAL OF MICROBIOLOGICAL METHODS, vol. 107, 12 October 2014 (2014-10-12), pages 98 - 105, XP029098802, ISSN: 0167-7012, DOI: 10.1016/J.MIMET.2014.09.008
- HALASA R. ET AL: "Comparison of fluorescence optical respirometry and microbroth dilution methods for testing antimicrobial compounds. Supplementary", JOURNAL OF MICROBIOLOGICAL METHODS, 12 October 2014 (2014-10-12), pages 98 - 105, XP055828694, Retrieved from the Internet <URL:https://ars.els-cdn.com/content/image/1-s2.0-S0167701214002863-mmc1.pdf> [retrieved on 20210729]
- ELMAR SCHM?LZLIN ET AL: "An Optical Multifrequency Phase-Modulation Method Using Microbeads for Measuring Intracellular Oxygen Concentrations in Plants", BIOPHYSICAL JOURNAL, vol. 89, no. 2, 1 August 2005 (2005-08-01), AMSTERDAM, NL, pages 1339 - 1345, XP055269787, ISSN: 0006-3495, DOI: 10.1529/biophysj.105.063453

## Beschreibung

Die Erfindung betrifft ein Verfahren zum respirometrischen Nachweis von Bakterien und zur Bestimmung der antibakteriellen Wirksamkeit antibiotischer Substanzen mittels auf Lumineszenz basierender Oxymetrie nach dem Oberbegriff des Patentansprüchs 1. Ein derartiges Verfahren wird zum Nachweis von Infektionen mit Bakterien und zur Bestimmung der Wirksamkeit von Antibiotika gegen diese Bakterien, also für eine Antibiotika-Resistenzbestimmung, genutzt. Ergebnis dieser Bestimmung ist ein Antibiogramm, das die antibakterielle Wirksamkeit verschiedener Substanzen auf in der untersuchten Probe enthaltene Bakterien angibt. Anhand dieses Ergebnisses kann dann ein geeignetes Antibiotikum oder eine geeignete Kombination mehrerer Antibiotika für die Therapie eines mit diesen Bakterien infizierten Patienten ausgewählt werden.

Ein im Stand der Technik im großen Umfang durchgeführtes Standardverfahren bei der Suche nach einem geeigneten Antibiotikum, also bei der Antibiotika-Resistenzbestimmung, besteht in der Vermehrung von Bakterien in einem geeigneten Nährmedium und der Bestimmung der Wirksamkeit eines Antibiotikums anhand der Geschwindigkeit, mit der sich die Bakterien bei Anwesenheit des Antibiotikums auf diesem Nährmedium vermehren. Ein großer Nachteil dieses bekannten Verfahrens ist jedoch, dass es zeitaufwändig in einem Labor durchgeführt werden muss und dass das Ergebnis der Resistenzbestimmung daher in der Regel erst nach einem Zeitraum von ein bis drei Tagen vorliegt. In der Praxis kann mit dem Einsatz von Antibiotika oftmals nicht so lange gewartet werden, so dass Patienten daher oftmals schon vor Vorliegen des Ergebnisses mit empirisch als wahrscheinlich wirksam ausgewählten Antibiotika behandelt werden. Ein Therapieerfolg stellt sich dabei jedoch nicht mit Sicherheit ein. Außerdem erhöhen sich durch die oftmals nicht optimale Wirksamkeit die Therapiekosten und die Wahrscheinlichkeit, dass Nebenwirkungen und Unverträglichkeiten beim Patienten auftreten und dass resistente Bakterienstämme erzeugt werden.

Ein anderes bekanntes Verfahren zu Antibiotika-Resistenzbestimmung basiert auf der respirometrischen Messung des aeroben bakteriellen Stoffwechsels mittels Sauerstoffelektroden. Dieses Verfahren ermöglicht zwar die schnellere Bereitstellung von Ergebnissen, erfordert jedoch einen hohen personellen Aufwand und eine große Probenmenge und konnte sich aus diesen Gründen nicht durchsetzen.

Bei einem anderen bekannten Verfahren, welches auf Sauerstoffbestimmung mittels Lumineszenz basiert, sind zwar der personelle Aufwand und die Probenmenge verringert. Jedoch erfordern diese Verfahren eine lange Messzeit von mindestens einer halben Stunde bis zu 12 Stunden. Für die schnelle Routinediagnostik sind diese Verfahren daher nicht geeignet und konnten sich daher nicht als Alternative zu dem eingangs genannten Verfahren im Labor durchsetzen.

Derartige Verfahren sind beispielsweise veröffentlicht in "O'Mahony, Fiach C. et al. (2006) Rapid High-Throughput Assessment of Aerobic Bacteria in Complex Samples by Fluorescence-Based Oxygen Respirometry. Applied and Environmental Microbiology, 72 No. 2, 1279-1287" und "Jasionek, G. et al. (2013) Rapid detection and respirometric profiling of aerobic bacteria on panels of selective media. Journal of Applied Microbiology 114, 423-432" und "Hatasa, R. et al. (2014) Comparison of fluorescence optical respirometry and microbroth dilution methods for testing antimicrobial compounds. Journal of Microbiological Methods, 107, 98-105".

Aus anderen Zusammenhängen prinzipiell bekannt sind darüber hinaus Verfahren zur Sauerstoffbestimmung mittels Lumineszenz, bei denen das anregende Licht mit unterschiedlichen Modulationsfrequenzen moduliert wird.

Zu nennen ist hier beispielsweise "Schmälzlin, E. et al. (2005) An Optical Multifrequency Phase-Modulation Method Using Microbeads for Measuring Intracellular Oxygen Concentrations in Plants. Biophysical Journal 89, 1339-1345".

In WO 2013/053381 A1 sind ein Verfahren und eine Vorrichtung zur Bestimmung einer Stoffkonzentration mittels Fluoreszenzspektroskopie offenbart. Dabei wird eine Probe mit Licht in zwei zueinander unterschiedlichen Modulationsfrequenzen bestrahlt, wobei die Intensität des Lichts moduliert wird. Mittels eines Detektors wird nachfolgend die Phasenlage einer von der Probe ausgesandten Fluoreszenzstrahlung bestimmt. Die detektierte Fluoreszenzstrahlung wird für jede Modulationsfrequenz hinsichtlich ihrer Phasenlage relativ zu einer jeweiligen ersten Referenzphasenlage mittels einer Auswerteelektronik ausgewertet.

Der Erfindung liegt die Aufgabe zugrunde, ein Verfahren anzugeben, mittels dem der Nachweis einer bakteriellen Infektion und die Bestimmung der antibakteriellen Wirksamkeit antibiotischer Substanzen in kurzer Zeit und mit einem geringen personellen Aufwand möglich sind.

Die Erfindung löst diese Aufgabe mit einem Verfahren nach dem Patentanspruch 1. Vorteilhafte Ausführungsformen der Erfindung sind in den Unteransprüchen angegeben.

Bei einem Verfahren zum respirometrischen Nachweis von Bakterien und zur Bestimmung der antibakteriellen Wirksamkeit antibiotischer Substanzen mittels auf Lumineszenz basierender Oxymetrie, wobei Anregungsmittel in wenigstens einem Messdurchgang mehrere Proben, welche jeweils wenigstens einen Luminophor enthalten und Anteile von einer auf Bakterien zu testenden Flüssigkeit enthalten und zumindest teilweise wenigstens eine antibiotische Substanz enthalten, mit Licht, welches den Luminophor anregt, bestrahlen und wobei Messmittel die daraufhin vom Luminophor ausgehende Lumineszenzantwort messen, ist erfindungswesentlich vorgesehen, dass die Probe mit Mikropartikeln, insbesondere Mikrokugeln, hergestellt wird, wobei die Mikropartikel den Luminophor enthalten und Radikalfänger aufweisen, die Singulett-Sauerstoff abfangen, und eine Größe im Bereich von 1 µm bis 100 µm aufweisen, dass die Anregungsmittel die Amplitude des anregenden Lichts mit wenigstens zwei unterschiedlichen Modulationsfrequenzen, insbesondere periodisch, modulieren und dass die Messmittel den Phasenwinkel zwischen dem anregenden Licht und der daraufhin vom Luminophor ausgehenden Lumineszenzantwort messen.

Der Luminophor ist ein Sensorfarbstoff, der zunächst in fester Form vorliegt. Die Mikropartikel müssen und sollen nicht in Lösung übergehen. Die Lumineszenz erfolgt an den Mikropartikeln. Der Sauerstoffgehalt wird also direkt an den Mikropartikeln gemessen.

Die Radikalfänger sind chemische Verbindungen, insbesondere Antioxidantien, die reaktive Radikale abfangen. Dank der Radikalfänger wird einer Schädigung der Bakterien durch Radikale wie dem Singulett-Sauerstoff und damit einer Verfälschung der Messergebnisse entgegengewirkt.

Die Erfindung liefert in sehr kurzer Zeit zuverlässige Ergebnisse zur Wirksamkeit antibiotischer Substanzen bei Proben, welche eine auf Bakterien zu testende Flüssigkeit enthalten. Vorzugsweise werden in jedem Messdurchlauf mehrere Testzyklen durchlaufen, wobei die Probe in jedem Testzyklus beleuchtet wird, um nachfolgend die Lumineszenzantwort zu messen. Die Proben können vor Ort in der Nähe des Patienten untersucht werden und müssen nicht in ein Labor verbracht werden. Dadurch kann sehr schnell getestet werden, welche antibiotischen Substanzen ein Bakterienwachstum verlangsamen und gegebenenfalls unterbinden können. Unmittelbar darauf kann ein Patient, von dem die Bakterien in der zu testenden Flüssigkeit stammen, mit einem als wirksam getesteten Antibiotikum behandelt werden. Je höher die Sauerstoffkonzentration ist, umso geringer ist die zeitliche Verzögerung der Lumineszenzantwort. Bakterieller Sauerstoffverbrauch in der Probe bewirkt also über die Abnahme der Sauerstoffkonzentration eine Zunahme der zeitlichen Verzögerung der Lumineszenzantwort. Aufgrund der Messung für mindestens zwei verschiedene Modulationsfrequenzen kann eine bei der Messung unerwünschte Untergrundlumineszenz, die insbesondere von der in der Probe enthaltenen Flüssigkeit, von einer in der Probe enthaltenen Nährlösung und von einem Behältnis für die Probe wie einer Wellplatte oder Mikrotiterplatte ausgeht, quantifiziert und dadurch von der vom Luminophor ausgehenden Lumineszenz abgetrennt werden. Dank der mindestens zwei Modulationsfrequenzen kann die Sauerstoffkonzentration damit selbst in Anwesenheit störender lumineszierender Aromaten in der Probe bestimmt werden.

Für das Messen ist insbesondere vorgesehen, dass die Messmittel den Phasenwinkel zwischen dem anregenden Licht und der Lumineszenzantwort mit Hilfe der mit den unterschiedlichen Modulationsfrequenzen modulierten Amplitude des anregenden Lichts mittels der für unterschiedliche Zeiten ermittelten Intensität der Lumineszenzantwort messen. Alternativ ermitteln die Messmittel den Phasenwinkel mittels einer analogen elektrischen Schaltung.

Eine beispielhafte Vorrichtung weist vorzugsweise eine Recheneinheit auf. Die Recheneinheit ist dazu ausgebildet und bei dem erfindungsgemäßen Verfahren ist vorzugsweise vorgesehen, dass die Recheneinheit aus den gemessenen Phasenwinkeln einer Vielzahl von Messungen den Sauerstoffabfall in den Proben pro Zeiteinheit, insbesondere unter Berücksichtigung einer eingestellten oder geschätzten Temperatur der Proben, als Maß für die Stoffwechseltätigkeit der Bakterien ermittelt. Beispielsweise wird eine Unterlage unter den Proben mit einer eingestellten Leistung beheizt oder derart geregelt beheizt, dass sich eine gewünschte Temperatur an dieser Unterlage einstellt, welche zu der eingestellten oder geschätzten Temperatur an den Proben führt. Die tatsächliche Temperatur an den Proben ist dabei vorzugsweise identisch mit der eingestellten oder geschätzten Temperatur oder weicht alternativ vorzugsweise weniger als 0,5 °C, weiter bevorzugt weniger als 0,2 °C, besonders bevorzugt weniger als 0,1 °C, von der eingestellten oder geschätzten Temperatur ab. Die gewünschte Temperatur, die hierbei eingestellt wird, liegt vorzugsweise im Bereich von 35 °C bis 42 °C oder 37 °C bis 42 °C, beträgt also in etwa Körpertemperatur. Die Temperatur ist dabei für alle Proben im Wesentlichen identisch, wobei Abweichungen der Temperatur der Proben untereinander vorzugsweise weniger als 0,5 °C, weiter bevorzugt weniger als 0,2 °C und besonders bevorzugt weniger als 0,1 °C betragen.

Bei einer vorteilhaften Ausführungsform des erfindungsgemäßen Verfahrens ist vorgesehen, dass die Messmittel im selben Messdurchgang Messungen an mehreren Proben, welche Anteile von der gleichen auf Bakterien zu testenden Flüssigkeit enthalten, durchführen. Auf diese Weise können innerhalb kurzer Zeiträume Messergebnisse zur Wirksamkeit unterschiedlicher antibiotischer Substanzen in den Proben erhalten werden. Auch Messungen an mehreren Proben mit den gleichen antibiotischen Substanzen sind möglich, um die statistische Aussagekraft der Ergebnisse zu erhöhen. Insbesondere ist hierbei vorgesehen, dass die Messmittel im selben Messdurchgang Messungen an mehreren Proben, welche die gleichen antibiotischen Substanzen enthalten, durchführen. Mit mehreren im selben Messdurchgang durchgeführten Messungen an Replikaten reduzieren sich die Standardfehler und damit die notwendige Messzeit für eine statistisch saubere Bestimmung des Sauerstoffabfalls pro Zeiteinheit. Alternativ oder zusätzlich werden bei einer bevorzugten Ausführungsform im selben Messdurchgang Messungen an mehreren Proben, welche unterschiedliche antibiotische Substanzen enthalten, durchgeführt. Die Messungen können in folgenden Messdurchgängen mit denselben oder unterschiedlichen antibiotischen Substanzen fortgeführt werden. In jedem Messdurchgang werden bevorzugt mehrere Messungen an jeder Probe durchgeführt, wobei die Probe für jede Messung jeweils kurz beleuchtet wird.

Die Messungen im selben Messdurchgang erfolgen entweder nacheinander oder, um eine weitere Beschleunigung des Verfahrens zu erhalten, teilweise gleichzeitig. Insbesondere ist dabei vorgesehen, dass die Messmittel mehrere Messungen an den Proben im selben Messdurchgang gleichzeitig mittels mehrerer gleichartiger Sensoren durchführen. Die Messmittel weisen hierfür die mehreren gleichartigen Sensoren auf. Vorzugsweise werden dabei mehrere Messungen an Proben, die unterschiedliche antibiotische Substanzen enthalten, gleichzeitig durchgeführt. Proben mit denselben antibiotischen Substanzen sind dabei bevorzugt über die Mikrotiterplatte verteilt angeordnet, um Positionseffekte statistisch durch Mittelung minimieren zu können. Alternativ oder zusätzlich werden bevorzugt mehrere Messungen an Proben, die dieselben antibiotischen Substanzen enthalten, gleichzeitig durchgeführt.

Die Proben, welche im selben Messdurchgang untersucht werden sollen, werden zuvor vorzugsweise in räumlicher Nähe und festen Abständen zueinander platziert. Bei einer bevorzugten Ausführungsform der Erfindung ist vorgesehen, dass die im selben Messdurchgang zu untersuchenden Proben auf einer Mikrotiterplatte hergestellt werden, wobei mehrere auf der Mikrotiterplatte angeordnete Näpfchen, welche insbesondere Kohlenhydrate mit oder ohne die antibiotischen Substanzen enthalten, mit je einem Anteil der Flüssigkeit, welche auf Bakterien zu testen ist, befüllt werden. Vorzugsweise ist dabei vorgesehen, dass die Näpfchen bereits vor dem Zugeben der Flüssigkeit die antibiotischen Substanzen enthalten. Die antibiotischen Substanzen liegen hierfür insbesondere in fester Form vor. In fester Form sind die antibiotischen Subtanzen für längere Zeit lagerfähig. Die Näpfchen können daher bereits eine Zeit vor dem Zugeben der Flüssigkeit hergestellt werden, was bei der Herstellung der Proben Zeit spart und den Aufbau der Vorrichtung vereinfacht.

Zum Befüllen der Näpfchen mit der Flüssigkeit zur Herstellung der Proben gibt es die Möglichkeit, die Proben zu bewegen oder Mittel zum Befüllen, wie eine Spritze, zu bewegen. Auch eine Kombination beider Bewegungen ist möglich und bei dem erfindungsgemäßen Verfahren bevorzugt. Insbesondere ist bei dem erfindungsgemäßen Verfahren vorgesehen, dass zum reihenweisen Befüllen der Näpfchen mit der Flüssigkeit, welche auf Bakterien zu testen ist, mehrmals nacheinander Fördermittel, insbesondere durch Bewegen eines Tisches, auf dem die Mikrotiterplatte aufliegt, die Mikrotiterplatte in einer Förderrichtung bewegen, so dass eine nächste zu befüllende Reihe von Näpfchen unter einer Spritze, insbesondere Pipette, eines Einfüllsystems angeordnet wird, und jeweils nachfolgend ein senkrecht zur Förderrichtung bewegbarer Schlitten des Einfüllsystems die Spritze senkrecht zur Förderrichtung über die in der jeweiligen Reihe angeordneten Näpfchen bewegt und das Einfüllsystem durch die Spritze die Näpfchen der jeweiligen Reihe nacheinander befüllt.

Bei einer hierzu alternativen Ausführungsform des erfindungsgemäßen Verfahrens ist vorgesehen, dass zum reihenweisen Befüllen der Näpfchen mit der Flüssigkeit, welche auf Bakterien zu testen ist, mehrmals nacheinander Fördermittel, insbesondere durch Bewegen eines Tisches, auf dem die Mikrotiterplatte aufliegt, die Mikrotiterplatte in einer Förderrichtung bewegen, so dass eine nächste zu befüllende Reihe von Näpfchen unter mehreren, insbesondere acht, in Reihe senkrecht zur Förderrichtung angeordneten Spritzen, insbesondere Pipetten, eines Einfüllsystems angeordnet wird, und das Einfüllsystem jeweils nachfolgend durch die Spritzen die Näpfchen der jeweiligen Reihe gleichzeitig befüllt. Hierdurch kann das Befüllen beschleunigt werden.

In der Spritze ist vorzugsweise ein Rührwerk des Einfüllsystems angeordnet, welches die Flüssigkeit in der Spritze fortlaufend durchmischt. Dadurch sind die Proben vergleichbar zusammengesetzt, insbesondere im Hinblick auf die Konzentration von Bakterien in der Flüssigkeit.

Vor Herstellung der Proben, also insbesondere vor Zugabe eines Anteils der auf Bakterien zu testenden Flüssigkeit, sind die antibiotischen Substanzen vorzugsweise als Feststoff, insbesondere gebunden in Kohlenhydraten (Sacchariden), in den Näpfchen angeordnet. Die derart vorbereiteten Näpfchen können über einen längeren Zeitraum gelagert werden und daher bereits vor Bereitstellung der auf Bakterien zu untersuchenden Flüssigkeit vorbereitet werden. Weiter ist vorzugsweise vorgesehen, dass in den Näpfchen enthaltene magnetische Rührstäbe das Lösen der antibiotischen Substanzen in der Flüssigkeit, welche auf Bakterien zu testen ist, unterstützen.

Vorzugsweise werden auch die Proben ohne antibiotische Substanzen mit Kohlenhydraten hergestellt. Die in den Näpfchen als Feststoff angeordneten oder anderweitig hinzugefügten Kohlenhydrate enthalten in diesem Fall keine antibiotischen Substanzen. Die Proben mit den antibiotischen Substanzen und die Vergleichsproben ohne antibiotische Substanzen werden vorzugsweise mit der gleichen Menge an Kohlenhydraten hergestellt. Dadurch wird eine vorteilhafte Vergleichbarkeit der Messergebnisse sichergestellt.

Verschiedene antibiotische Substanzen weisen oft ein zueinander unterschiedliches Lösungsverhalten auf. Vorteilhaft ist es daher, insbesondere für eine zeitnahe Messung nach dem Befüllen der Näpfchen mit der Flüssigkeit, wenn die vergleichsweise schwerer löslichen antibiotischen Substanzen zuerst Flüssigkeit abbekommen. Vorzugsweise sieht das erfindungsgemäße Verfahren daher vor, dass unterschiedliche antibiotische Substanzen mit unterschiedlichem Lösungsverhalten in der Reihenfolge ihres Lösungsverhaltens in den Näpfchen auf der Mikrotiterplatte angeordnet sind und dass die Näpfchen in dieser Reihenfolge, beginnend mit den vergleichsweise schlechter löslichen antibiotischen Substanzen, mit der Flüssigkeit, welche auf Bakterien zu testen ist, befüllt werden.

Vorteilhafterweise temperiert eine Heizeinheit zum Lösen der antibiotischen Substanzen die Proben oder einen Tisch, auf dem die Mikrotiterplatte aufliegt, insbesondere durch Beheizen des Tisches, auf eine Temperatur im Bereich von 37 °C bis 42 °C. Bei einer Temperatur in diesem Bereich, welche auch den Wachstumsbedingungen in vivo, also im lebendigen Organismus, entspricht, lösen sich antibiotische Substanzen besser und metabolisieren Bakterien Sauerstoff in der Regel schneller als bei vergleichsweise niedrigerer Temperatur.

Für die an den Proben durchzuführen Messungen weisen die Anregungsmittel wenigstens eine Lichtquelle, vorzugsweise genau eine Lichtquelle, auf. Die Lichtquelle ist vorzugsweise eine LED oder ein Laser. Die Messungen an den einzelnen Proben erfolgen vorzugsweise nacheinander. Zur Platzierung der Anregungsmittel, insbesondere des Lasers, sowie zur Platzierung eines Sensors der Messmittel über oder an einer jeweiligen Probe gibt es, ähnlich wie beim Befüllen der Näpfchen, die Möglichkeit, die Proben zu bewegen oder die Anregungsmittel und den Sensor zu bewegen. Auch eine Kombination beider Bewegungen ist möglich und bei dem erfindungsgemäßen Verfahren bevorzugt.

Bei einer besonderen Ausführungsform des erfindungsgemäßen Verfahrens ist vorgesehen, dass die Messmittel in einem vorgeschalteten Messdurchgang Messungen an mehreren Proben, die in Näpfchen auf einer separaten Mikrotiterplatte angeordnet sind und die nicht mit antibiotischen Substanzen bestückt sind, durchführen. Diese Messungen erfolgen, um erst einmal festzustellen, ob überhaupt Bakterien, die mittels antibiotischer Substanzen bekämpft werden sollten, in den Proben enthalten sind. Bevorzugt ist daher weiter vorgesehen, dass nur dann, wenn hierbei anhand der gemessenen Lumineszenzantwort ein auf Bakterienwachstum deutender Sauerstoffverbrauch ermittelt wird, nachfolgend die Proben mit den antibiotischen Substanzen hergestellt und in einem nachfolgenden Messdurchgang Messungen mit diesen Proben durchgeführt werden. Alternativ können die Messungen an den Proben mit den antibiotischen Substanzen und an den Vergleichsproben ohne antibiotische Substanzen, jedoch auch im selben Messdurchgang, insbesondere bei Anordnung der Proben und Vergleichsproben in Näpfchen auf derselben Mikrotiterplatte, durchgeführt werden.

Weitere Ausführungsformen der Erfindung ergeben sich aus den Patentansprüchen, aus den Zeichnungen und aus der nachfolgenden Beschreibung eines in den Zeichnungen dargestellten besonders bevorzugten Ausführungsbeispiels der Erfindung. In den Zeichnungen zeigen:
- Fig. 1:: Teile einer Vorrichtung zur respirometrischen Bestimmung der antibakteriellen Wirksamkeit antibiotischer Substanzen gemäß einem ersten Ausführungsbeispiel der Erfindung in perspektivischer Ansicht; und
- Fig. 2:: Teile einer Vorrichtung zur respirometrischen Bestimmung der antibakteriellen Wirksamkeit antibiotischer Substanzen gemäß einem zweiten Ausführungsbeispiel der Erfindung in perspektivischer Ansicht.

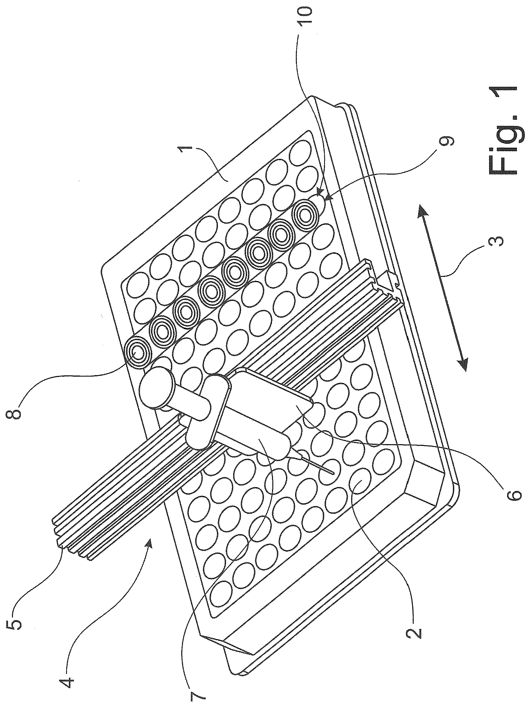

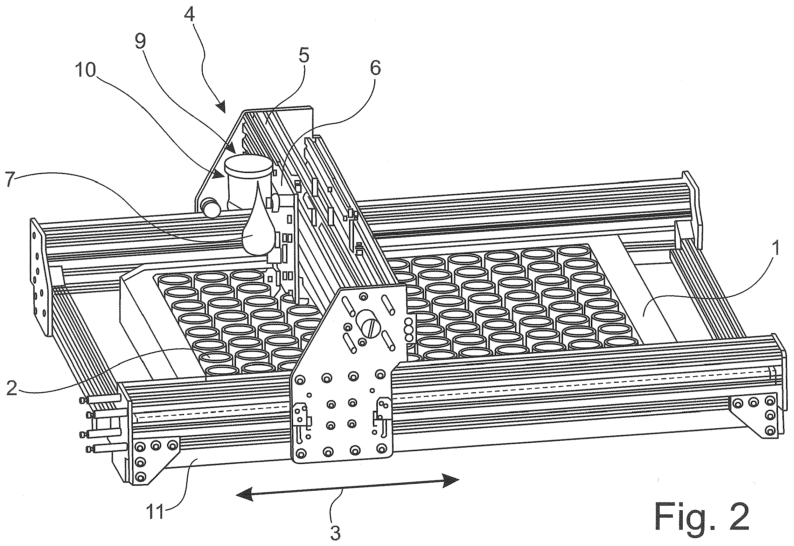

In den Figuren 1 und 2 sind jeweils Teile von Vorrichtungen zur respirometrischen Bestimmung der antibakteriellen Wirksamkeit antibiotischer Substanzen mittels auf Lumineszenz basierender Oxymetrie dargestellt. Mit den Vorrichtungen kann jeweils das erfindungsgemäße Verfahren zur respirometrischen Bestimmung der antibakteriellen Wirksamkeit antibiotischer Substanzen mittels auf Lumineszenz basierender Oxymetrie durchgeführt werden. Hierfür wird die Sauerstoffkonzentration über Proben, welche jeweils wenigstens einen Luminophor enthalten und Anteile von einer auf Bakterien zu untersuchenden Flüssigkeit enthalten und zumindest teilweise eine antibiotische Substanz enthalten, untersucht.

Die zu untersuchenden Proben werden auf einer Mikrotiterplatte 1 bereitgestellt. Die Mikrotiterplatte 1 weist in beiden Ausführungsbeispielen jeweils 96 Vertiefungen auf. Jede Vertiefung bildet ein Näpfchen 2 zur Aufnahme jeweils einer Probe aus. In den Figuren ist beispielhaft nur ein Näpfchen 2 bezeichnet. Die Näpfchen 2 sind in zwölf Reihen und acht Spalten angeordnet.

Vor Herstellung der Proben sind alle oder zumindest mehrere Näpfchen 2 bereits mit der antibiotischen Substanz bestückt. Die antibiotischen Substanzen sind hierfür in Kohlenhydraten (Sacchariden) gebunden. Einzelne Näpfchen 2 können für Kontrollzwecke, ob überhaupt Bakterien in den Proben enthalten sind, ohne antibiotische Substanzen ausgerüstet sein. Die Flüssigkeit mit den darin enthaltenen Bakterien wird in die Näpfchen 2 und dabei gegebenenfalls auf die Kohlenhydrate mit den antibiotischen Substanzen gegeben.

In jedem Näpfchen 2 sind darüber hinaus bereits vor dem Befüllen mit der Flüssigkeit ein Luminophor und ein magnetischer Rührstab enthalten. Die gegebenenfalls vorhandene antibiotische Substanz löst sich in der Flüssigkeit, was durch eine magnetisch angetriebene Drehbewegung des jeweiligen Rührstabes unterstützt wird. Dabei lösen sich die Kohlenhydrate, in denen die antibiotischen Substanzen angeordnet sind, in der zu testenden Flüssigkeit auf, geben dadurch die antibiotischen Substanzen frei und sind nachfolgend vorzugsweise Nährmedium für die in der Flüssigkeit enthaltenen Bakterien.

Zum Befüllen der Näpfchen 2 mit der Flüssigkeit wird in der Vorrichtung des Ausführungsbeispiels von Figur 1 die Mikrotiterplatte 1 in einer parallel zu den Reihen der Näpfchen 2 auf der Mikrotiterplatte 1 angeordneten Förderrichtung 3 unter ein in Figur 1 dargestelltes Einfüllsystem 4 gefahren. Das Einfüllsystem 4 weist einen an einer Schiene 5 geführten und senkrecht zur Förderrichtung 3 bewegbaren Schlitten 6 auf. Am Schlitten 6 ist eine Spritze 7 mit einem elektrisch ansteuerbaren Ventil und einem in der Spritze 7 angeordneten Rührwerk befestigt. Die Spritze kann somit zunächst durch Bewegung des Schlittens 6 über einem Näpfchen 2 einer Reihe positioniert werden und dann durch Bewegung des Schlittens 6 nacheinander über allen weiteren Näpfchen 2 in der jeweiligen Reihe positioniert werden. Durch kontrolliertes Öffnen und Schließen des Ventils wird jeweils eine definierte Menge der zu untersuchenden Flüssigkeit aus der Spritze 7 in das Näpfchen 2 gegeben. Das Rührwerk sorgt dabei für eine gleichmäßige Zusammensetzung der Flüssigkeit in den herzustellenden Proben, insbesondere für eine vergleichbare Menge darin enthaltener Bakterien.

Die Messungen der Sauerstoffkonzentration erfolgen bei der Vorrichtung von Figur 1 mittels einer Sensorzeile 8, welche Anregungsmittel 9 und Messmittel 10 aufweist. Die Anregungsmittel 9 weisen für jedes Näpfchen 2 in einer jeweiligen Reihe jeweils eine, insbesondere halbleiterbasierte, Lichtquelle, insbesondere eine LED oder einen Laser, zueinander gleicher Bauart auf. Jede Lichtquelle erzeugt Licht einer bestimmten Wellenlänge mit periodischer Intensitätsänderung. Die Messmittel 10 weisen für jedes Näpfchen 2 in dieser Reihe jeweils einen Sensor, insbesondere Lichtsensor, gleicher Bauart auf. Die Bestimmung des Phasenwinkels der Lumineszenzantwort sowie der Intensität erfolgt für mindestens zwei Anregungsfrequenzen. Über jedem Näpfchen 2 der Reihe sind dabei jeweils eine Lichtquelle und jeweils ein Sensor, insbesondere Lichtsensor, positioniert. Dadurch sind gleichzeitige Messungen an allen in den Näpfchen 2 der Reihe befindlichen Proben möglich. Für Messungen an einer folgenden Reihe wird die Mikrotiterplatte 1 in der Förderrichtung 3 verfahren.

Die Vorrichtung weist eine nicht dargestellte Recheneinheit auf, welche den Sauerstoffabfall in den Proben pro Zeiteinheit berechnet. Die Mikrotiterplatte 1 kann auf einen in Figur 1 nicht dargestellten, insbesondere beheizbaren, Tisch aufgelegt und mit Hilfe des Tisches relativ zum Einfüllsystem 4 und zur Sensorzeile 8 in Förderrichtung 3 bewegt werden. Dabei wird entweder der Tisch bewegt oder die Schiene 5 über dem Tisch verfahren. Ferner ist ein nicht dargestellter Mikrocomputer vorgesehen, der Schrittmotoren zur Bewegung des Tisches oder der Schiene 5 in Förderrichtung 3, sowie zur Bewegung des Schlittens 6 quer zur Förderrichtung 3 steuert. Die Recheneinheit führt eine statistische Auswertung des zeitlichen Verlaufs der Lumineszenzantwort für jede einzelne Probe oder für jede Gesamtheit gleichartiger Proben durch. In den gleichartigen Proben, die vorzugsweise über die Mikrotiterplatte verteilt angeordnet sind, sind dabei gleiche Kombinationen aus antibiotischer Substanz und zu testender Flüssigkeit angeordnet. Zur Darstellung von Ergebnissen sind vorzugsweise ein in Figur 1 nicht dargestelltes Display und eine Schnittstelle zu einem Netzwerk vorgesehen, über welches Ergebnisse beispielsweise an ein vom behandelnden Arzt einsehbares Endgerät übertragen werden können.

Figur 2 zeigt Teile der Vorrichtung gemäß einem zur Vorrichtung von Figur 1 alternativen und bevorzugten Ausführungsbeispiel, bei welchem die Anregungsmittel 9 und die Messmittel 10 nur zur Messung an jeweils einer Probe zur selben Zeit ausgebildet sind. Insbesondere weisen die Anregungsmittel 9 nur eine einzige Lichtquelle und die Messmittel 10 nur einen einzigen Sensor, insbesondere Lichtsensor, auf. Die Anregungsmittel 9 und die Messmittel 10 sind dabei zur selben Zeit über derselben Probe angeordnet und können senkrecht zur Förderrichtung 3 bewegt werden. Hierfür sind die Anregungsmittel 9 und die Messmittel 10 zusammen mit dem Einfüllsystem 4 am Schlitten 6 angeordnet und an der Schiene 5 senkrecht zur Förderrichtung 3 bewegbar. Das Einfüllsystem 4 mit der Spritze 7 ist in Figur 2 nur schematisch als Tropfen dargestellt, kann jedoch entsprechend der Darstellung in Figur 1 ausgebildet sein. Gleiche Bezugszeichen in den Figuren bezeichnen gleiche oder funktional einander entsprechende Teile.

Die Mikrotiterplatte 1 liegt in Figur 2 auf dem bereits in der Beschreibung zu Figur 1 genannten und hier erstmals bezeichneten Tisch 11 auf. Die Schiene 5 und der Tisch 11 mit der Mikrotiterplatte 1 können in Förderrichtung 3 relativ zueinander verfahren werden, insbesondere um eine andere Reihe von Näpfchen 2 unter der Spritze 7, dem Laser und dem Sensor zu positionieren.

## Patentansprüche

1. Verfahren zum respirometrischen Nachweis von Bakterien und zur Bestimmung der antibakteriellen Wirksamkeit antibiotischer Substanzen mittels auf Lumineszenz basierender Oxymetrie, wobei Anregungsmittel (9) in wenigstens einem Messdurchgang mehrere Proben, welche jeweils wenigstens einen Luminophor enthalten und Anteile von einer auf Bakterien zu testenden Flüssigkeit enthalten und zumindest teilweise wenigstens eine antibiotische Substanz enthalten, mit Licht, welches den Luminophor anregt, bestrahlen und wobei Messmittel (10) die daraufhin vom Luminophor ausgehende Lumineszenzantwort messen,
**dadurch gekennzeichnet,**
**dass** die Probe mit Mikropartikeln, insbesondere Mikrokugeln, hergestellt wird, wobei die Mikropartikel den Luminophor enthalten und Radikalfänger aufweisen, die Singulett-Sauerstoff abfangen, und eine Größe im Bereich von 1 µm bis 100 µm aufweisen, dass die Anregungsmittel (9) die Amplitude des anregenden Lichts mit wenigstens zwei unterschiedlichen Modulationsfrequenzen, insbesondere periodisch, modulieren und dass die Messmittel (10) den Phasenwinkel zwischen dem anregenden Licht und der daraufhin vom Luminophor ausgehenden Lumineszenzantwort messen.

2. Verfahren nach Anspruch 1, **dadurch gekennzeichnet, dass** die Messmittel (10) den Phasenwinkel zwischen dem anregenden Licht und der Lumineszenzantwort mit Hilfe der mit den unterschiedlichen Modulationsfrequenzen modulierten Amplitude des anregenden Lichts mittels der für unterschiedliche Zeiten ermittelten Intensität der Lumineszenzantwort messen.

3. Verfahren nach einem der Ansprüche 1 und 2, **dadurch gekennzeichnet, dass** eine Recheneinheit aus den gemessenen Phasenwinkeln einer Vielzahl von Messungen den Sauerstoffabfall in den Proben pro Zeiteinheit, insbesondere unter Berücksichtigung einer eingestellten oder geschätzten Temperatur der Proben, als Maß für die Stoffwechseltätigkeit der Bakterien ermittelt.

4. Verfahren nach einem der vorhergehenden Ansprüche, **dadurch gekennzeichnet, dass** die Messmittel (10) im selben Messdurchgang Messungen an mehreren Proben, welche Anteile von der gleichen auf Bakterien zu testenden Flüssigkeit enthalten, durchführen.

5. Verfahren nach einem der vorhergehenden Ansprüche, **dadurch gekennzeichnet, dass** die Messmittel (10) im selben Messdurchgang Messungen an mehreren Proben, welche die gleichen antibiotischen Substanzen oder unterschiedliche antibiotische Substanzen enthalten, durchführen.

6. Verfahren nach Anspruch 5, **dadurch gekennzeichnet, dass** die Messmittel (10) mehrere Messungen an den Proben im selben Messdurchgang gleichzeitig mittels mehrerer gleichartiger Sensoren durchführen.

7. Verfahren nach einem der vorhergehenden Ansprüche, **dadurch gekennzeichnet, dass** die im selben Messdurchgang zu untersuchenden Proben auf einer Mikrotiterplatte (1) hergestellt werden, wobei mehrere auf der Mikrotiterplatte (1) angeordnete Näpfchen (2), welche insbesondere Kohlenhydrate mit oder ohne die antibiotischen Substanzen enthalten, mit einem Anteil der Flüssigkeit, welche auf Bakterien zu testen ist, befüllt werden.

8. Verfahren nach Anspruch 7, **dadurch gekennzeichnet, dass** zum reihenweisen Befüllen der Näpfchen (2) mit der Flüssigkeit, welche auf Bakterien zu testen ist, mehrmals nacheinander Fördermittel, insbesondere durch Bewegen eines Tisches (11), auf dem die Mikrotiterplatte (1) aufliegt, die Mikrotiterplatte (1) in einer Förderrichtung (3) bewegen, so dass eine nächste zu befüllende Reihe von Näpfchen (2) unter einer Spritze (7), insbesondere Pipette, eines Einfüllsystems (4) angeordnet wird, und jeweils nachfolgend ein senkrecht zur Förderrichtung (3) bewegbarer Schlitten (6) des Einfüllsystems (4) die Spritze (7) senkrecht zur Förderrichtung (3) über die in der jeweiligen Reihe angeordneten Näpfchen (2) bewegt und das Einfüllsystem (4) durch die Spritze (7) die Näpfchen (2) der jeweiligen Reihe nacheinander befüllt.

9. Verfahren nach Anspruch 7, **dadurch gekennzeichnet, dass** zum reihenweisen Befüllen der Näpfchen (2) mit der Flüssigkeit, welche auf Bakterien zu testen ist, mehrmals nacheinander Fördermittel, insbesondere durch Bewegen eines Tisches (11), auf dem die Mikrotiterplatte (1) aufliegt, die Mikrotiterplatte (1) in einer Förderrichtung (3) bewegen, so dass eine nächste zu befüllende Reihe von Näpfchen (2) unter mehreren, insbesondere acht, in Reihe senkrecht zur Förderrichtung (3) angeordneten Spritzen (7), insbesondere Pipetten, eines Einfüllsystems (4) angeordnet wird, und das Einfüllsystem (4) jeweils nachfolgend durch die Spritzen (7) die Näpfchen (2) der jeweiligen Reihe gleichzeitig befüllt.

10. Verfahren nach einem der Ansprüche 8 und 9, **dadurch gekennzeichnet, dass** ein in der Spritze (7) angeordnetes Rührwerk des Einfüllsystems (4) die Flüssigkeit in der Spritze (7) fortlaufend durchmischt.

11. Verfahren nach einem der Ansprüche 7 bis 10, **dadurch gekennzeichnet, dass** die antibiotischen Substanzen vor Herstellung der Proben als Feststoff, insbesondere gebunden in Kohlenhydraten, in den Näpfchen (2) angeordnet sind und dass in den Näpfchen (2) enthaltene magnetische Rührstäbe das Lösen der antibiotischen Substanzen in der Flüssigkeit, welche auf Bakterien zu testen ist, unterstützen.

12. Verfahren nach Anspruch 11, **dadurch gekennzeichnet, dass** unterschiedliche antibiotische Substanzen mit unterschiedlichem Lösungsverhalten in der Reihenfolge ihres Lösungsverhaltens in den Näpfchen (2) auf der Mikrotiterplatte (1) angeordnet sind und dass die Näpfchen (2) in dieser Reihenfolge, beginnend mit den vergleichsweise schlechter löslichen antibiotischen Substanzen, mit der Flüssigkeit, welche auf Bakterien zu testen ist, befüllt werden.

13. Verfahren nach einem der Ansprüche 11 und 12, **dadurch gekennzeichnet, dass** eine Heizeinheit die Proben auf eine Temperatur im Bereich von 37 °C bis 42 °C temperiert oder einen Tisch (11), auf dem die Mikrotiterplatte (1) aufliegt, insbesondere durch Beheizen des Tisches (11), auf eine Temperatur im Bereich von 37 °C bis 42 °C temperiert.

14. Verfahren nach einem der vorhergehenden Ansprüche, **dadurch gekennzeichnet, dass** die Messmittel (10) im selben Messdurchgang Messungen an mehreren Proben, die in Näpfchen (2) auf einer separaten Mikrotiterplatte (1) angeordnet sind und die nicht mit antibiotischen Substanzen bestückt sind, durchführen und dass nur dann, wenn hierbei anhand der gemessenen Lumineszenzantwort ein auf Bakterienwachstum deutender Sauerstoffverbrauch ermittelt wird, nachfolgend die Proben mit den antibiotischen Substanzen hergestellt und Messungen mit diesen Proben durchgeführt werden.

## Claims

1. A method for the respirometric detection of bacteria and for the determination of the antibacterial efficacy of antibiotic substances by means of luminescence-based oximetry wherein, in at least one measuring cycle, excitation means (9) irradiate a plurality of samples with light, which samples each contain at least one luminophore and fractions of a liquid to be tested for bacteria and at least in part contain at least one antibiotic substance, which light excites the luminophore, and wherein measuring means (10) measure the luminescence response thereupon emanating from the luminophore,
**characterized in that**
the sample is prepared with microparticles, in particular microbeads, wherein the microparticles contain the luminophore and radical scavengers which scavenge singlet oxygen and have a size in the range of 1 µm to 100 µm, **in that** the excitation means (9) modulate the amplitude of the excitation light with at least two different modulation frequencies, in particular periodically, and **in that** the measuring means (10) measure the phase angle between the excitation light and the luminescence response thereupon emanating from the luminophore.

2. The method as claimed in claim 1, **characterized in that** the measuring means (10) measure the phase angle between the excitation light and the luminescence response with the aid of the amplitude of the excitation light modulated with the different modulation frequencies by means of the intensity of the luminescence response determined for different times.

3. The method as claimed in one of claims 1 and 2, **characterized in that** a processing unit determines the oxygen decrease in the samples per unit time from the measured phase angles of a plurality of measurements, in particular taking a preset or estimated temperature of the samples into consideration, as a measure of the metabolic activity of the bacteria.

4. The method as claimed in one of the preceding claims, **characterized in that** in the same measuring cycle, the measuring means (10) carry out measurements on a plurality of samples which contain fractions of the same liquid to be tested for bacteria.

5. The method as claimed in one of the preceding claims, **characterized in that** in the same measuring cycle, the measuring means (10) carry out measurements on a plurality of samples which contain identical antibiotic substances or different antibiotic substances.

6. The method as claimed in claim 5, **characterized in that** the measuring means (10) carry out a plurality of measurements on the samples simultaneously in the same measuring cycle by means of a plurality of identical sensors.

7. The method as claimed in one of the preceding claims, **characterized in that** the samples to be investigated in the same measuring cycle are prepared on a microtitre plate (1), wherein a plurality of cells (2) disposed on the microtitre plate (1), which cells in particular contain carbohydrates with or without the antibiotic substances, are filled with a fraction of the liquid which is to be tested for bacteria.

8. The method as claimed in claim 7, **characterized in that** in order to fill the cells (2) with the liquid which is to be tested for bacteria in a row-by-row manner several times one after the other, conveying means move the microtitre plate (1) in a conveying direction (3), in particular by moving a table (11) on which the microtitre plate (1) is positioned, so that a row of cells (2) which is to be filled next becomes disposed under a syringe (7), in particular a pipette, of a filling system (4), and a carriage (6) of the filling system (4) which can be moved perpendicularly to the conveying direction (3) moves the syringe (7) perpendicularly to the conveying direction (3) over the cells (2) disposed in the respective row respectively one after the other and the filling system (4) fills the cells (2) of the respective row one after the other via the syringe (7).

9. The method as claimed in claim 7, **characterized in that** in order to fill the cells (2) with the liquid which is to be tested for bacteria in a row-by-row manner several times one after the other, conveying means move the microtitre plate (1) in a conveying direction (3), in particular by moving a table (11) on which the microtitre plate (1) is positioned, so that a row of cells (2) which is to be filled next becomes disposed under a plurality of, in particular eight, syringes (7), in particular pipettes, of a filling system (4), which syringes are disposed in a row perpendicular to the conveying direction (3), and respectively one after the other, the filling system (4) simultaneously fills the cells (2) of the respective row via the syringes (7).

10. The method as claimed in one of claims 8 and 9, **characterized in that** a stirring unit of the filling system (4) disposed in the syringe (7) continuously mixes the liquid in the syringe (7).

11. The method as claimed in one of claims 7 to 10, **characterized in that** prior to the preparation of the samples, the antibiotic substances are disposed in the cells (2) as a solid, in particular bound into carbohydrates, and **in that** magnetic stirring rods contained in the cell (2) support the dissolution of the antibiotic substances in the liquid which is to be tested for bacteria.

12. The method as claimed in claim 11, **characterized in that** different antibiotic substances with different solubility behaviours are disposed in the cells (2) on the microtitre plate (1) in the sequence of their solubility behaviours and **in that** the cells (2) are filled with the liquid which is to be tested for bacteria in this sequence, beginning with the comparatively lower solubility antibiotic substances.

13. The method as claimed in one of claims 11 and 12, **characterized in that** a heating unit controls the temperature of the samples to a temperature in the range of 37°C to 42°C or controls the temperature of a table (11) on which the microtitre plate (1) is positioned to a temperature in the range of 37°C to 42°C, in particular by heating the table (11).

14. The method as claimed in one of the preceding claims, **characterized in that** in the same measuring cycle, the measuring means (10) carry out measurements on a plurality of samples which are disposed in cells (2) on a separate microtitre plate (1) and which are not provided with antibiotic substances, and **in that** the samples with the antibiotic substances are subsequently prepared and measurements are carried out with these samples only if an oxygen consumption which indicates bacterial growth is then determined thereby with the aid of the measured luminescence response.

## Revendications

1. Procédé destiné à détecter des bactéries par respirométrie et à déterminer l'efficacité antibactérienne de substances antibiotiques par oxymétrie basée sur la luminescence, consistant à exposer au cours d'au moins un passage de mesure, à l'aide de moyens d'excitation (9), plusieurs échantillons, dont chacun contient au moins un luminophore et contient une proportion d'un liquide à analyser pour la présence de bactéries et contient au moins partiellement au moins une substance antibiotique, à de la lumière qui excite ledit luminophore, et des moyens de mesure (10) mesurant alors la réponse de luminescence émise par le luminophore,
**caractérisé en ce que**
ledit échantillon est préparé avec des microparticules, notamment des microbilles, lesdites microparticules contenant ledit luminophore et comportant des agents anti-radicalaires qui neutralisent de l'oxygène singulet, et leur taille étant comprise entre 1 µm et 100 µm, que les moyens d'excitation (9) viennent moduler l'amplitude de la lumière d'excitation au moyen d'au moins deux fréquences de modulation, notamment de manière périodique, et que les moyens de mesure (10) viennent mesurer l'angle de phase entre la lumière d'excitation et la réponse de luminescence alors émise par le luminophore.

2. Procédé selon la revendication 1, caractérisé en qu'à partir de l'intensité de la réponse de luminescence telle que déterminée à différents moments au fil du temps, les moyens de mesure (10) viennent mesurer l'angle de phase entre la lumière d'excitation et la réponse de luminescence, en s'aidant de l'amplitude de la lumière d'excitation qui est modulée avec différentes fréquences de modulation.

3. Procédé selon l'une des revendications 1 et 2, **caractérisé en ce qu'**une unité de calcul détermine, à partir des angles de phase mesurés lors d'une pluralité de mesures, la baisse du taux d'oxygène dans les échantillons par unité de temps en tant qu'indicateur pour l'activité métabolique des bactéries, en prenant notamment en compte une température réglée ou estimée des échantillons.

4. Procédé selon l'une des revendications précédentes, **caractérisé en ce que** les moyens de mesure (10) viennent réaliser, dans un même passage de mesure, des mesures sur plusieurs échantillons contenant des proportions du même liquide à analyser pour la présence de bactéries.

5. Procédé selon l'une des revendications précédentes, **caractérisé en ce que** les moyens de mesure (10) viennent réaliser, dans un même passage de mesure, des mesures sur plusieurs échantillons contenant les mêmes substances antibiotiques ou différentes substances antibiotiques.

6. Procédé selon la revendication 5, **caractérisé en ce que** les moyens de mesure (10) effectuent, lors d'un même passage de mesure, plusieurs mesures sur les échantillons simultanément à l'aide de plusieurs capteurs d'un même type.

7. Procédé selon l'une des revendications précédentes, **caractérisé en ce que** les échantillons destinés à être analysés dans un même passage de mesure sont préparés sur une plaque microtitre (1), plusieurs puits (2) qui sont disposés sur la plaque microtitre (1) et qui contiennent notamment des glucides avec ou sans lesdites substances antibiotiques, étant remplis avec une proportion du liquide à analyser pour la présence de bactéries.

8. Procédé selon la revendication 7, **caractérisé en ce que**, pour remplir les rangés de puits (2) avec ledit liquide à analyser pour la présence de bactéries, la plaque microtitre (1) est déplacée à plusieurs reprises selon un sens de transport (3) par des moyens de transport, en déplaçant notamment la table (11) sur laquelle repose la plaque microtitre (1), faisant en sorte que la prochaine rangée de puits (2) à remplir soit disposée au-dessous d'une seringue (7), s'agissant plus particulièrement d'une pipette, d'un système de remplissage (4), pour ensuite déplacer à chaque instance, à l'aide d'un chariot (6) qui est mobile dans un sens perpendiculaire audit sens de transport (3) et qui fait partie du système de remplissage (4), la seringue (7) perpendiculairement au sens de transport (3) jusqu'à ce qu'elle se trouve au-dessus des puits (2) disposés dans la rangée concernée, permettant ainsi au système de remplissage (4) de remplir, au moyen de la seringue (7), successivement les puits (2) de la rangée concernée.

9. Procédé selon la revendication 7, **caractérisé en ce que**, pour remplir les rangés de puits (2) avec ledit liquide à analyser pour la présence de bactéries, la plaque microtitre (1) est déplacée à plusieurs reprises selon un sens de transport (3) par des moyens de transport, en déplaçant notamment la table (11) sur laquelle repose la plaque microtitre (1), faisant en sorte que la prochaine rangée de puits (2) à remplir soit disposée au-dessous d'une pluralité de seringues (7), notamment au nombre huit, s'agissant plus particulièrement de pipettes, qui sont disposées en série perpendiculairement au sens de transport (3) et qui font partie d'un système de remplissage (4), ce qui permet ensuite à chaque instance au système de remplissage (4) de remplir, au moyen des seringues (7), simultanément les puits (2) de la rangée concernée.

10. Procédé selon l'une des revendications 8 et 9, **caractérisé en ce qu'**un organe d'agitation, qui fait partie du système de remplissage (4) et se trouve au sein de la seringue (7), assure que le liquide dans la seringue (7) est continuellement mélangé.

11. Procédé selon l'une des revendications 7 à 10, **caractérisé en ce que**, préalablement à la préparation des échantillons, lesdites substances antibiotiques sont disposées dans les puits (2) sous une forme solide, notamment liées à des glucides, et que des barreaux d'agitation magnétiques, contenus dans les puits (2), favorisent la dissolution des substances antibiotiques dans le liquide à analyser pour la présence de bactéries.

12. Procédé selon la revendication 11, **caractérisé en ce que** différentes substances antibiotiques ayant différents comportements en ce qui concerne leur solubilité sont disposées selon l'ordre de leur solubilité dans les puits (2) sur la plaque microtitre (1), et que les puits (2) sont remplis avec le liquide à analyser pour la présence de bactéries en suivant cet ordre, en commençant par les substances antibiotiques relativement peu solubles.

13. Procédé selon l'une des revendications 11 et 12, **caractérisé en ce qu'**une unité de chauffage augmente la température des échantillons de manière à ce qu'elle soit comprise entre 37 °C et 42 °C ou bien augmente la température d'une table (11) sur laquelle la plaque microtitre (1) est posée, en chauffant notamment la table (11) à une température comprise entre 37 °C et 42 °C.

14. Procédé selon l'une des revendications précédentes, **caractérisé en ce que** les moyens de mesure (10) viennent effectuer, dans un même passage de mesure, des mesures sur plusieurs échantillons qui sont disposés dans des puits (2) sur une plaque microtitre (1) séparée et qui sont dépourvus de substances antibiotiques, et que seulement si la réponse de luminescence qui y est mesurée permet d'identifier une consommation d'oxygène indiquant un développement bactérien, on procède ensuite à la préparation d'échantillons pourvus de substances antibiotiques et à la réalisation de mesures avec ces échantillons.
